# EUROPEAN PATENT APPLICATION

(11) **EP 4 241 597 A1**
(43) Date of publication of application: **13.09.2023**
(21) Application number: 21887975.7
(22) Date of filing: 18.10.2021
(51) Int. Cl.: A41D 19/00, A41D 19/015, A61F 5/00, A61H 1/00, A61F 13/00, A61F 13/10

(54) **HAND-AND-WRIST ACCESSORY DEVICE FOR PEOPLE THAT FACILITATES EXTENSION MOVEMENTS**

(30) Priority: 04.11.2020 CL 20202852
(71) Applicant: Gogo Labs SPA, Las Condes Santiago (CL)
(72) Inventor: GORUP VALENZUELA, Cristian Eduardo, Las Condes Santiago (CL); GODOY SANTANDER, Diego Antonio, Maipú Santiago (CL)
(74) Representative: ABG Intellectual Property Law, S.L.
(86) International application number: PCT/CL2021/050103
(87) International publication number: WO 2022/094736

(57) **Abstract**

Disclosed is a hand-and-wrist accessory device formed by an elasticated glove, wherein the ends of the fingers and the end of the wrist comprise an internal elasticated silicone layer that acts as a mechanical securing element for securing to the skin, these securing elements being connected to rigid structures connected to elastic elements, transmitting elastic energy from the wrist to a central floating piece on the dorsal carpus, where it is distributed to each finger terminal, the elastic elements being guided by rigid pieces disposed in the paths extending from the central piece to the finger terminals, to ensure the correct alignment thereof. These external elements have reciprocal securing elements to generate continuity from the wrist terminal to the finger or digit terminals. Cushioned elements are disposed on the palm to redistribute pressure in the wrist areas.

## Description

### FIELD OF THE INVENTION

The present application relates to a (dynamic) orthotic glove that facilitates finger and wrist extension movements in typing, this accessory specifically performing the function of preventing painful symptoms in the hand and wrist by means of padded elastic structures with the function of reducing fatigue and damage to extensor muscles of the fingers, hand and wrist by redistributing pressures at anatomical points such as the carpal tunnel, hypothenar, and thenar of the hand.

### BACKGROUND

At present, injuries due to repetitive movements are common in typing and manufacturing professions. With the massification of technology, these pathologies and symptoms have shown an increase across age and gender, and additionally there is a variable use of repetitive movements in leisure time that increases the possibility of generating pathologies such as carpal tunnel, tendinitis, trigger finger, and tenosynovitis.

Conventional therapies for these complications are immobilization by orthotics, neuromuscular taping, pharmacotherapy, and kinesiological or physiotherapeutic rehabilitation when an acute or chronic pathological phenomenon has been diagnosed, and in some cases, a surgical solution is reached. The prevention of these injuries is achieved by means of periodic stretching of the hand and wrist muscles, and ergonomic postures and accessories.

One of the therapeutic principles of neuromuscular taping is the mechanical facilitation of muscles by means of elastic bands that are positioned on the path of a muscle or set of muscles related to the action movement produced by them.

There are several hand and upper limb accessories to protect these structures from different variables, from temperature to extreme mechanical forces. There are also those serving ornamental and comfort purposes, on the other hand, there are orthotics for the treatment of hand, wrist and forearm pathologies which are designed for the treatment of such pathologies and prevent their recurrence in response to a diagnosis, maintaining a principle of graduated restricted mobilization and/or immobilization of the anatomical structures so that these comply with the healing process without further complexity.

One of the most representative solutions in typing work environments is to follow ergonomic standards that are designed to avoid health complications of this type, with workstations generally having accessories for the purposes of preventing and protecting against hand, wrist and forearm injuries due to repetitive movements and poor postures. Even so, these interventions are not entirely effective because they are passive, are not integrated into a single device for routine use and are dependent on the participation of the users.

The methods mentioned and currently used have the additional drawback of being primarily solutions in response to a diagnosis or advanced stage of deterioration, and those that are for prevention are by means of passive elements, requiring guidance and monitoring by a professional, reducing the effectiveness of the therapeutic objective and affordability of techniques for prevention.

It is therefore apparent that there is a need for an element that performs the prevention of painful symptoms in repetitive typing work that is independent of the installation, guidance, and monitoring of a professional on site, with structures that are dynamic with respect to the movement of the hand and passive at pressure points, protecting the anatomical structures related to tendon pathologies and nerve compression.

Among the patents investigated, the most similar is patent US2005/223469A1, also for prevention with respect to repetitive movements, the difference with the present invention being that this patent focuses on bending movements and on console type video game controls, its principle being the use of terminal agents for the protection of muscles and mechanical facilitation.

The solution proposed in the present application is based on the objective of facilitating movement and reducing the mechanical loading of finger extension, resembling the widely used therapeutic principle of neuromuscular taping (USPTO no. 3523859) and selectively protecting pressure areas by means of passive cushioned elements, ensuring the protection of the anatomical structures related to tendon and nerve pathologies.

In this context, the incorporation of this accessory, which may comply with the therapeutic objectives of prevention independent of the installation, guidance and correction of a healthcare professional, is vital for increasing the affordability of elements for prevention in typing work and opens up a new concept for different areas of work using repetitive hand movements, protecting the health of workers and preventing restrictions on their daily life activities.

### DESCRIPTION OF THE INVENTION

The accessory consists of a glove with cut out fingers which, in the upper part, has elastic external structures that exert mechanical support on the path of the tendons of the common extensor muscle of the fingers for the purpose of facilitating the extension of fingers from the flexed finger position when typing on keys, in addition to cushioned structures on the thenar areas, hypothenar areas, pads, radius and ulnar areas, in the terminal areas of the fingers and wrist, is secured to the skin by means of silicone fabric on the inside of the glove, for the purpose of providing external structures with a mechanical securing element required for facilitating extension movements.

The glove is formed by breathable, hypoallergenic, elastane (spandex), non-toxic, washable, anti-fungal silicone microfibers in the distal areas of the fingers and wrist, being of a single color, of various colors or textures, shapes, and motifs, where summer or winter fabrics are also used as well.

The advantages of this accessory reside in the accessibility and ease of use of a tool for prevention without the need for installations and professional guidance, increasing comfort while at the same time releasing a load from anatomical structures, with a service life that is longer than that of neuromuscular taping, being adaptable to an elastic load, and having pieces that are easy to replace, being a reusable orthotic for the prevention of tendon pathologies, thereby preventing occupational desertion.

Considering what has been set out above, it would be beneficial to develop an accessory for alleviating or reducing the painful symptoms associated with the mechanical loading of the extensor muscles and tendons of the hand in typing positions for prolonged periods of time, performing the function of preventing muscle and tendon pathologies.

In summary, the present invention implements a glove, with elastic structures along the path of the extensor tendons (dorsal part of the hand) of the fingers, hand, and wrist, pressure dissipation structures on the thenar, hypothenar, carpal tunnel and metacarpal areas.

### BRIEF DESCRIPTION OF THE FIGURES

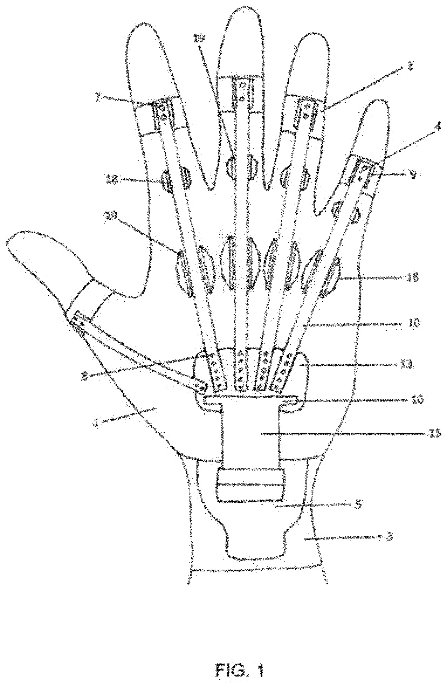

Figure 1 is an illustration showing a hand with the glove of the present invention in a top view.

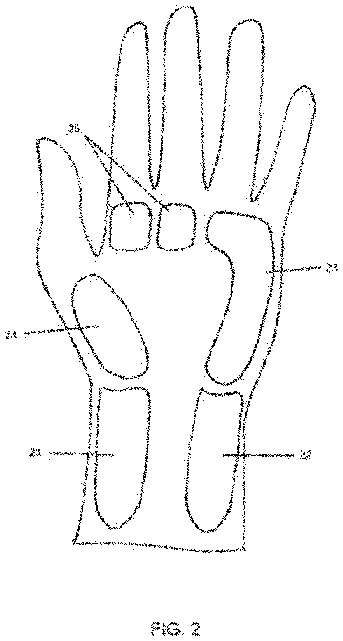

Figure 2 is an illustration showing a hand with the glove of the present invention in a bottom or palmar view.

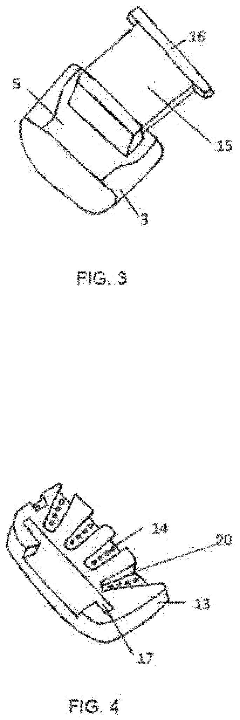

Figure 3 depicts a top perspective view of the support and wrist securing element of the glove.
Figure 4 depicts a top perspective view of the carpal or central securing piece of the present invention.

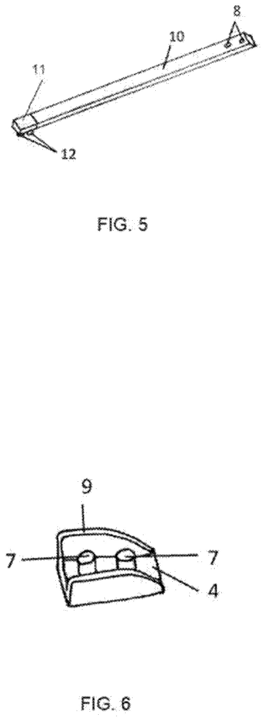

Figure 5 depicts a top perspective view of the elastic element and the securing mechanisms of the finger and carpal tendon support system.
Figure 6 depicts a top perspective view of the securing mechanisms of the distal finger tendon support system.

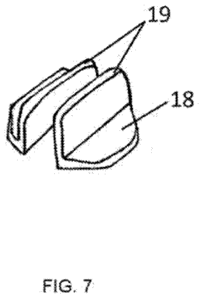

Figure 7 depicts a top perspective view of the guide element for guiding the elastic elements along the extension path.

### DETAILED DESCRIPTION OF THE INVENTION

The (dynamic) orthotic glove that facilitates finger extension in typing work is made up of a fitted elasticated glove (1) that covers from the proximal phalanges of the thumb and medial phalanges of the fingers to the wrist joint, constituting the main body of the device where the other functional elements are housed.

The elasticated glove (1) is formed by breathable, hypoallergenic, spandex, non-toxic, washable, anti-fungal silicone microfibers in the distal areas of the fingers and wrist for the purpose securing to the skin of the hand and wrist by adhesion and compression, there being positioned in these areas rigid finger terminals (4) and a wrist terminal (15), in a wrist securing area (5) and finger securing area (2).

The rigid finger terminal (4) has two side tabs (9) for the purpose of centering the path of elastic elements (10) and has two finger connection bosses (7) which reciprocally work with elastic perforations (8) in the elastic elements (10), the elastic perforations (8) of the exotendon elastic elements (10) performing the function of generating a finger extending force when the hand is in a flexed position over a mouse or an analog computer keyboard, where the proximal part of this element has a connection terminal (11) having two extruded elastic bosses (12) which reciprocally connect with two central piece perforations (14) of a central piece (13) for the purpose of being mechanically secured and generating a tension graduation system depending on the selection of coupling points, generating a connectivity with the elastic structures disposed from the wrist securing element (5) to the rigid finger terminals (4), being attached in the central piece (13) on the dorsal part of the hand, said central piece (13) defining five slots (20) each with at least two central piece perforations (14).

The wrist securing element (5) has a certain height from its securing point with the terminals for the elastic elements (10) and a joining piece (15) for the purpose of preventing friction between the aforementioned elastic pieces and the textile material of the glove.

The objective of the wrist securing element (5) adhered on the surface of the wrist (3) which internally comprises silicone is to project the joining piece (15) from an removable articulation to the central piece (13) in the area of the carpus of the glove of the hand, being secured to same by means of the articulation comprising a male articulation piece (16) with a shape extending onto a female articulation piece (17) designed to be reciprocally coupled and thereby generate continuity between external pieces.

The objective of the finger exotendon elastic structures or elastic elements (10) is to generate an elastic force towards finger extension and to restrict hand and finger flexing in typing positions and in positions where work is done on personal computers, or PCs, where the finger exotendon elastic structures are guided along a specific path of each tendon of the common extensor of the fingers by means of rigid guides (18) having two rigid guide side tabs (19) guiding the path of the elastic elements (10) over the metacarpals and the proximal phalanges of the hand and thumb, where the modular design allows replacement of these elastic elements (10) so as to customize them, adjust the tension, or perform maintenance.

The palm comprises external cushioned elements or pads disposed in anatomical positions for the purpose of releasing loads from areas where tendons, muscles, and nerves are located. The pads of the radial area (21) and the pads of the ulnar area (22) are disposed with a separation between them to favor loading on areas of the path of the radius and of the ulna, together with pads in the thenar area (24), the hypothenar area (25), and on the area of the palmar digital protuberance (25), being disposed to release pressure loading on the path of the carpal tunnel and hollow center of the palm where the main flexor tendons are located and to favor ventilation.

### List of parts:

- 1: elasticated glove
- 2: finger securing area
- 3: wrist
- 4: rigid finger terminals
- 5: wrist securing area
- 6: finger connection bosses
- 7: elastic perforations
- 8: side tabs
- 9: elastic elements
- 10: connection terminal
- 11: extruded elastic bosses
- 12: central piece
- 13: central piece perforations
- 14: joining piece
- 15: male articulation piece
- 16: female articulation piece
- 17: rigid guides
- 18: rigid guide side tabs
- 19: slots
- 20: cushioned elements in the radial area of the hand
- 21: cushioned elements in the ulnar area
- 22: cushioned elements in the hypothenar area
- 23: cushioned elements in the thenar area
- 24: cushioned elements in the palmar digital protuberance area

## Claims

1. A hand-and-wrist accessory device for people that facilitates extension movements, that allows the alleviation or reduction of the painful symptoms associated with the mechanical loading of the extensor muscles and tendons of the hand in typing positions for prolonged periods of time, performing the function of preventing muscle and tendon pathologies, **characterized in that** it comprises:
- an elasticated glove (1), wherein the distal ends of its fingers and wrist define an elasticated silicone layer, that is secured to the skin by means of silicone fabric on the inside of the glove, there being positioned in these areas a number of rigid finger terminals (4) and a wrist terminal (15), in a wrist securing area (5) and finger securing area (2);
- in the area of the dorsal carpus bones of the hand in the glove there is included a central piece (13) in the area of the dorsal carpus bones of the hand which joins with the wrist terminal (15) by means of an articulation, said central piece (13) defining five slots (20) each with at least two central piece perforations (14);
- on each finger of the glove in the medial phalange of the fingers on the dorsal part, rigid finger terminals (4) are defined on the finger securing element (2), each one having two finger connection bosses (7);
- four rigid guides (18) between the area of the dorsal carpus bones of the hand and the knuckle area of the hand and four rigid guide pieces (18) in the knuckle area of the hand, with each rigid guide piece (18) following the direction of the fingers;
- five elastic elements (10), one for each finger, comprising at one end two extruded elastic bosses (12) which reciprocally connect with the central piece perforations (14) of the central piece (13), and at the other end elastic perforations (8), complementary with the finger connection bosses (7) of the rigid finger terminal (4), these connection means defining different tension graduation levels according to the position in which they are coupled;
- the elastic elements are installed between the central piece (13) and rigid finger terminals (9) by means of the bosses and perforations, furthermore guided by the rigid guides (18), extending along a path similar to the biomechanics of the common extensor tendons of the fingers, disposed in the paths extending from the central piece to the finger terminals, to ensure the correct alignment thereof, with the elastic element which corresponds to the thumb of the glove being connected only between the central piece (13) and the rigid finger terminal (9);
- pads or cushioned elements are disposed on the palm of the hand of the glove in the radial area of the hand (21), the ulnar area (22), the hypothenar area (23), the thenar area (24) and the area of the palmar digital protuberance (25) to redistribute pressure in the wrist areas.

2. The device that facilitates hand and wrist extension movements according to claim 1, **characterized in that** the glove (1) is formed by breathable, hypoallergenic, elastane (spandex), non-toxic, washable, anti-fungal silicone microfibers in the distal areas of the fingers and wrist, being of a single color, of various colors or textures, shapes, and motifs, where summer or winter fabrics are also used as well.

3. The device that facilitates hand and wrist extension movements according to claim 1, **characterized in that** the central piece (13), joining piece (15), guide piece (18), and rigid finger terminal (4) are manufactured from thermoplastic materials.

4. The device that facilitates hand and wrist extension movements according to claim 1, **characterized in that** the rigid finger terminal (4) has two side tabs (9) for the purpose of centering the path of the elastic element (10).

5. The device that facilitates hand and wrist extension movements according to claim 2, **characterized in that** the wrist securing element (5) has a certain height from its securing point with the terminals for the elastic elements (10) and a joining piece (15) for the purpose of preventing friction between the elastic pieces and the textile material of the glove.

6. The device that facilitates hand and wrist extension movements according to claim 1, **characterized in that** the articulation comprises a male articulation piece (16) with a shape extending onto a female articulation piece (17) designed to be reciprocally coupled.
